Europäisches Patentamt

European Patent Office

Office européen des brevets

(11) Publication number: 0 369 459
A2

## (12) EUROPEAN PATENT APPLICATION

(21) Application number: 89121296.1

(22) Date of filing: 17.11.89

(51) Int. Cl.⁵: C12P 7/64, C12P 9/00, A61K 37/22, //(C12P7/64, C12R1:91)

(30) Priority: 18.11.88 JP 292563/88

(43) Date of publication of application:
23.05.90 Bulletin 90/21

(84) Designated Contracting States:
DE FR GB

(71) Applicant: Sansho Seiyaku Co., Ltd.
26-7, Oike 2-chome
Onojo-shi Fukuoka-ken(JP)

Applicant: KABUSHIKI KAISHA HAYASHIBARA
SEIBUTSU KAGAKU KENKYUJO
2-3, Shinmoishii 1-chome
Okayama-shi Okayama-ken(JP)

Applicant: Mishima, Yutaka
4-32, Sowa-cho 1-chome Nada-ku
Kobe-shi Hyogo-ken(JP)

(72) Inventor: Mishima, Yutaka
4-32, Sowa-cho 1-chome, Nada-ku
Kobe-shi, Hyogo-ken(JP)
Inventor: Oyama, Yasuaki
15-16, Oike 2-chome
Onojo-shi, Fukuoka-ken(JP)
Inventor: Kurimoto, Masashi
28-5, Ifuku-cho 3-chome
Okayama-shi, Okayama-ken(JP)

(74) Representative: Patentanwälte Deufel- Schön-
Hertel- Lewald- Otto
Isartorplatz 6
D-8000 München 2(DE)

(54) Process for preparing melanogenic inhibitor, and pigmentation-lightening agent containing the same.

㊼ The present invention relates to a process for preparing a melanogenic inhibitor which is obtained by proliferating an established cell line from a warm-blooded animal in vitro, or by proliferating the cell line under the supply of a nutrient body fluid from a non-human warm-blooded animal, disrupting the proliferated cells and extracting a lipid component containing fatty acids and their derivatives. The invention also relates to a pigmentation-lightening agent comprising the lipid component containing the fatty acid and their derivatives extracted from the established cell line from a warm-blooded animal.

Xerox Copy Centre

# PROCESS FOR PREPARING MELANOGENIC INHIBITOR, AND PIGMENTATION-LIGHTENING AGENT CONTAINING THE SAME

## BACKGROUND OF THE INVENTION

### Field of the invention

The present invention relates to a process for extracting and preparing a lipid component containing fatty acids and their derivatives, the lipid component possessing melanogenic-inhibitory activity and existing in an established cell line from warm-blooded animals. The present invention also relates to a pigmentation-lightening agent containing the same.

### Description of the prior art

Conventionally, a great number of attempts have been conducted in order to produce a pigmentation-lightened skin and to prevent generation of chloasma and ephelis, by decreasing the level of melanin in the skin.

For example, cosmetics using vitamin C, glutathione and cysteine, those using quercetin as a flavone compound, (Japanese Patent Kokai No. Sho. 55-92305, Japanese Patent Kokai No. Sho. 57-14517) and those using myrisetin, fisetin, gutiscetin and rhamnetin (Japanese Patent Kokai No. Sho. 55-11410, Japanese Patent Kokai No. Sho. 55-35506) have been disclosed.

Principally, conventional melanogenesis-inhibitory substances are those which inhibit tyrosinase activity, and they have disadvantages such as instability and insufficient efficacy in producing a pigmentation-lightened skin via viable cells.

## SUMMARY OF THE INVENTION

An object of the present invention is to provide a process for preparing a novel melanogenic inhibitor to inhibit melanogenesis via use of function of viable cells and to provide a pigmentation-lightening agent containing the inhibitor as an effective component, without inhibiting tyrosinase activity.

The present inventors had investigated to obtain a substance which might inhibit melanogenesis via use of the function of viable cells and might have a pigmentation-lightening effect. As a result, the present inventors found that a novel melanogenic inhibitor which does not substantially inhibit tyrosinase activity, but exhibits melanogenic-inhibitory- and dopa-reaction-inhibitory-activities in a prescribed pigment cell, is obtainable by proliferating a transferable warm-blooded animal cell (referred to as "established cell line" in the present specification hereinafter), homogenizing the proliferated cells, and recovering the melanogenic inhibitor as a water-soluble substance from the resultant homogenate. Thus, they invented a process for preparing the substance and a pigmentation-lightening agent using the substance as an effective component (Japanese Patent Application No. Sho. 62-336470).

The present inventors further continued .this investigation. As a result, they found that a slightly water-soluble melanogenic inhibitor exists in the established cell line from warm-blooded animals, which exhibits a melanogenesis inhibitory activity without affecting tyrosinase activity, and also found that the above inhibitor is a substance containing fatty acids and their derivatives. Thus, they achieved this invention.

## DETAILED DESCRIPTION OF THE INVENTION

The present invention relates to a process for preparing a melanogenic inhibitor which is obtained by proliferating an established cell line from a warm-blooded animal in vitro, or by proliferating the cell line under the supply of a nutrient body fluid from a non-human warm-blooded animal, disrupting the proliferated cells and extracting a lipid component containing fatty acids and their derivatives. The invention also relates to a pigmentation-lightening agent comprising the lipid component as an effective component, which contains fatty acids and their derivatives extracted from the established cell line from a warm-blooded animal.

Any established cell line used as raw materials in the process according to the present invention can be freely employed insofar as it readily proliferates in vitro or proliferates while receiving a body fluid from a non-human warm-blooded animal, and it produces the melanogenic inhibitor. Example of such established cell lines include those derived from warm-blooded animals available from Dainippon Pharmaceutical Co., Ltd., Tokyo, Japan, or those available from American Type Culture Collection, 12301 Parklawn Drive Rockville, Maryland 20852-1776, and those established cell lines can be favorably selected.

These established cell lines may be proliferated in vitro, or in vivo under the supply of a nutrient body fluid from a non-human warm-blooded animal.

Preferably, the established cell lines are proliferated by directly implanting in the body of a non-human warm-blooded animal, or inoculating into a diffusion chamber for proliferation while receiving a nutrient body fluid of a non-human warm-blooded animal.

The melanogenic inhibitor can be obtained from the established cell lines proliferated in this manner, by disrupting the established cell lines, for example, by homogenization, ultrasonication and lyophilization, and by extracting the cell homogenate with chloroform, butanol, isopropanol, ether, acetone, ethanol, methanol and hexane and a mixed solution thereof.

Preferably, the cell homogenate is homogeneously mixed with acetone and homogenized thereinto about 24 hours. Subsequently, the resultant homogenate is filtered and the acetone is evaporated from the filtrate, followed by the addition of ethanol to the residue.

The substance which can be obtained by the process in accordance with the present invention is a lipid component which contains unsaturated fatty acids such as pentadecenoic acid ($C_{15:1}$), oleinoic acid ($C_{18:1}$), linoleic acid ($C_{18:2}$), linolenic acid ($C_{18:3}$) and eicosatetraenoic acid ($C_{20:4}$), and fatty acid derivatives such as glyceride or phospholipid thereof, slightly water-soluble but soluble in organic solvents such as methanol, ethanol, acetone and hexane.

The melanogenic inhibitory activity in a prescribed pigment cell is determined by the method described in Cancer Research, Vol.42, pp. 1994-2002 (1982).

B-16 cells ($4 \times 10^4$) derived from mouse melanoma is suspended in 10 ml Eagle's minimum essential medium (Grand Island Biological Co., Grand Island, N. Y.), supplemented with 10% fetal calf serum. The suspension is placed into a $25 cm^2$ Roux's flask, and cultured at $37^{\circ}$ C in the presence of 5% $CO_2$. The cultivation is continued for 5 days while replacing the culture medium with a fresh one supplemented with a melanogenic inhibitor specimen at the first- and third-day. After completion of the culture, the resultant cells are washed with phosphate buffer (pH 7.2) containing 0.8 w/v % saline to detach them from the inside wall of the Roux's flask by the addition of a solution containing trypsin and EDTA (ethylenediamine tetraacetic acid), collected on a filter paper, and dried. The resultant cells on the filter paper are subjected to densitometry to measure the reflection light at a wave length of 500 nm, followed by determination of the reflection absorbance (degree of darkness).

One unit of melanogenic inhibitory activity is defined as the amount of the melanogenic inhibitor which halves the reflection absorbance of B-16 cells without treatment of melanogenic inhibitor (control).

The product exhibited a melanogenic inhibitory activity of about 100 units/mg on the dry solid basis in a prescribed pigment cell and the yield was about 5000 units per 100g of the established cell line used.

The pigmentation-lightening agent of the present invention is used in the form of medicine such as injections, oral agents, external applications, and bath liquids and also in cosmetics such as emulsion, pack, cream and lotion.

Such pigmentation-lightening preparations are produced according to routine methods, by adding about 0.001 - 10,000 units/dose of the pigmentation-lightening agent to injections, oral agents, external applications, bath liquids, or cosmetics which contain bases, vehicles and if necessary, antioxidants and cyclodextrin.

The present invention provides a process for preparing a novel melanogenic inhibitor which does not substantially inhibit tyrosinase activity but exhibits melanogenic-inhibitory activity in a pigment cell, and a pigmentation-lightening agent containing the melanogenic inhibitor as its active ingredient.

Since the present melanogenic inhibitor has a strong melanogenesis inhibitory activity and exerts a high efficacy in realizing a pigmentation-lightened skin, the pigmentation-lightening agent in accordance with the invention can be used in pharmaceuticals, for example, injections, oral agents, external applications, bath liquids, bath salts, etc. and in cosmetics, for example, emulsion, pack, cream, etc. for the prevention and treatment of local and systemic chromatosis such as chloasma, ephelis, sunburn and addisonism.

The preparation of the melanogenic inhibitor according to the present invention will be explained by the

3

following examples in detail.

## BRIEF DESCRIPTION OF THE DRAWINGS

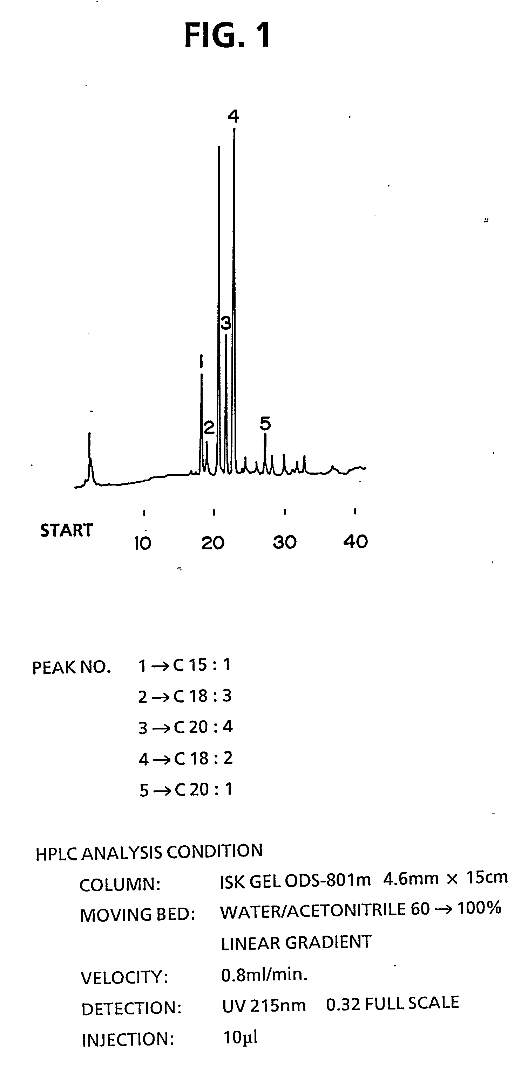

Fig. 1 is a graph showing the result of HPLC (high-performance liquid chromatography) analysis of the product obtained in Example A-1.

## DETAILED DESCRIPTION OF PREFERRED EMBODIMENTS

### Example A-1

Namalwa cells (ATCC CRL 1432) which were established cell lines derived from human leukemia was inoculated to the Eagle's minimal essential medium (pH 7.4) supplemented with 20% fetal calf serum, and subjected to conventional in vitro suspension culture at 37° C.

After completion of the culture, the resultant cells were centrifugally collected and homogenized in acetone for 24 hours. After soaking, the resultant homogenate was subjected to suction filtration and the filtrate was concentrated under reduced pressure to evaporate acetone. To the residue was added ethanol and after sufficient stirring, it was purified by membrane-filtration to yield a solution containing a melanogenic inhibitor.

HPLC (high-performance liquid chromatography) analysis of the product confirms that the product contains unsaturated fatty acids as shown in Fig. 1.

The product exhibited a melanogenic inhibitory activity of about 100 units/mg on the dry solid basis in a prescribed pigment cell and the yield was about 5000 units per 100g of the established cell line used.

The product can be used in pharmaceutical agents and cosmetics as a pigmentation-lightening agent and it exerts a high efficacy in realizing a pigmentation- lightened skin for prophylaxis and treatment of local or systemic chromatosis.

### Example B-1

#### Injections

One part by weight of a melanogenic inhibitor, obtained by the method in Example A-1, was dissolved in 200 parts by weight of physiological saline, and the mixture was membrane-filtered in the usual manner. Two milliliter of the filtrate were distributed into sterilized glass vials and hermetically sealed to obtain an injection.

### Example B-2

#### Tablet

Three parts by weight of a melanogenic inhibitor, obtained by the method in Example A-1, was homogeneously mixed with 82 parts by weight of maltose, 61 parts by weight of corn starch, and 4 parts by weight of sucrose fatty acid ester, and the mixture was tabletted in usual manner to obtain tablets, 150 mg weight each.

### Example B-3

#### Ointment

Five parts by weight of a melanogenic inhibitor, obtained by the method in Example A-1, was homogeneously mixed with 10.0 parts by weight of glycerine. The resultant mixture was added to a mixture comprising 10.0 parts by weight of vaseline, 10.0 parts by weight of Japan tallow, 10.0 parts by weight of lanolin, 14.5 parts by weight of sesame oil, and 0.5 parts by weight of Japanese mint oil, and the resultant mixture was homogeneously mixed to obtain an ointment.

Example B-4

Bath liquid

One part by weight of a melanogenic inhibitor, obtained by the method in Example A-1, was mixed with 80 parts by weight of ethanol, and 19 parts by weight of purified water together with small amounts of coloring-and flavoring-agents to obtain a bath liquid.

Example B-5

Emulsion

0.5 Part by weight of polyoxyethylene behenyl ether, 1.0 part by weight of polyoxyethylene sorbitol tetraoleate, 1.0 part by weight lipophilic glyceryl monostearate, 0.5 parts by weight of stearic acid, 0.5 parts by weight of behenyl alcohol, 1.0 part by weight of avocado oil and small amounts of vitamin E, antiseptic and flavoring agent were dissolved by heating in the usual manner. To the mixture was added 1.0 part by weight of a melanogenic inhibitor obtained by the method in Example A-1 and 5.0 parts by weight of 1,3-butylene glycol, and the resultant mixture was homogenized to yield a emulsion.

Example B-6

Pack

Two parts by weight of a melanogenic inhibitor, obtained by the method in Example A-1, was homogeneously mixed in the usual manner with 1.5 parts by weight of squalene, 0.5 parts by weight of hydrogenated castor oil, 4.0 parts by weight of glycerin, 15.0 parts by weight of polyvinyl alcohol, 10.0 parts by weight of ethanol, and 67 parts by weight of purified water to obtain a pack.

Example B-7

Cream

Two parts by weight of polyoxyethylene glycol monostearate, 5 parts by weight of glyceryl monostearate SE, 5 parts by weight of stearic acid, 1 part by weight of behenyl alcohol, 1 part by weight of liquid paraffine, 10 parts by weight of glyceryl trioctanoate and small amounts of antiseptic and flavoring agent were dissolved by heating in the usual manner. To the mixture was added 2 parts by weight of melanogenic inhibitor, obtained by the method in Example A-1, 5 parts by weight of 1,3-butylene glycol, and 75 parts by weight of purified water, and the resultant mixture was homogenized to obtain a cream.

Example B-8

Lotion

Two parts by weight of a melanogenic inhibitor obtained in Example A-1, was homogeneously mixed with one part by weight of hydrogenated castor oil, 15 parts by weight of ethanol, 0.1 parts by weight of citric acid, 0.3 parts by weight of sodium citrate, 4.0 parts by weight of butylene glycol, 0.5 parts by weight of sodium pyrrolidone carboxylate, small amounts of antiseptic and flavoring agent and 77 parts of purified water in the usual manner to produce a lotion.

### Example A-2

RPMI 1846 cells which (ATCC CCL 49) were established cell lines derived from hamster melanoma were subcutaneously implanted in hamsters which were then fed in the usual way. According to the method in Example A-1, the tumor masses, subcutaneously formed in the hamsters, were removed and homogenized. The resultant homogenate was purified by gel-filtration chromatography, concentrated and sterilized by heating, and membrane-filtered, after which the resultant product was dried under the reduced pressure to obtain an oily melanogenic inhibitor.

TLC (thin layer chromatography) analysis of the product confirms that the product contains fatty acids as shown in the following.

(1) TLC plate

To silicagel is added 60ml of 12.5% $AgNO_3$ and air-dried.

Developing solvent

Hexane : ether : acetic acid (60:40:1)

Coloring reagent

0.2% dibromofluorescein in ethanol solution

Confirmation

A spot is confirmed at aposition of Rf = 0.342 under UV spot.

(2) TLC plate

Silicagel Developing solvent

Hexane : ether : acetic acid (60:40:1)

Color reagent

Sulfuric acid : acetic acid (1:1)

Heating at 110° C for 5 minutes

Confirmation

Brown spots develop at a position of Rf = 0.34

The product exhibited a melanogenic inhibitory activity of about 150 units/mg on the oily basis and the yield was about 7000 units per 100g of the established cell line used.

The product can be used as a pigmentation-lightening agent in the form of medicine and cosmetics in the same way as in Example A-1 and exerts a high pigmentation-lightening efficacy in prophylaxis and treatment on local and systemic chromatosis.

### Experimental Example

Comparison of melanogenic inhibitory effects

According to the method for determining melanogenic inhibitory activity on pigment cells, 1 or 3 units of the melanogenic inhibitor obtained in Example A-1 and A-2 were added to various established cell lines from melanoma, i.e. B-16 cells from mouse melanoma, RPMI 1846 cells from hamster melanoma and Malme-3M cells from human melanoma, and then the melanogenic inhibitory effects on these cells were compared mutually.

No melanogenic inhibitor was added to controls.

Results are shown in Table 1.

Table 1

| Experimental group | Added amount | Effects |
|---|---|---|
| Example A-1 | 1 unit | + |
| | 3 units | + + |
| Example A-2 | 1 unit | + |
| | 3 units | + + |
| Control | | - |
| Note: | . | |
| -: black<br>+: clearly white<br>+ +: remarkable white | | |

As shown in Table 1, any experimental group to which the melanogenic inhibitor was added showed stronger melanogenic inhibitory activity and demonstrated greater pigmentation-lightening effects, dose-dependently, compared with the activity shown in the controls.

Vitamin C, glutathione and cysteine, all of which are tyrosinase-inhibiting agents, did not show any melanogenic inhibitory activity.

## Claims

1. A process for preparing melanogenic inhibitor comprising:
either allowing an established cell line from a warm-blooded animal to proliferate in vitro or allowing the established cell line to proliferate under the supply of a nutrient body fluid of a non-human warm-blooded animal, and
recovering from the resultant cell a lipid component containing at least either of fatty acid and its derivative.

2. The process of claim 1, wherein said established cell line is a member selected from the group consisting of Namalwa cell (ATCC CRL 1432) and RPMI 1846 cell (ATCC CCL 49).

3. The process of claim 1, wherein said fatty acid is a member selected form the group consisting of pentadecenoic acid ($C_{15:1}$), oleinoic acid ($C_{18:1}$), linolenic acid ($C_{18:2}$), linolenic acid ($C_{18:3}$), eicosatetraenoic acid ($C_{20:4}$), and mixtures thereof.

4. The process of claim 1, wherein said fatty acid derivative is a member selected from the group consisting of glycerides and phospholipids of pentadecenoic acid ($C_{15:1}$), oleinoic acid ($C_{18:1}$), linolenic acid ($C_{18:2}$), linolenic acid ($C_{18:3}$), eicosatetraenoic acid ($C_{20:4}$), and mixtures thereof.

5. The process of claim 1, wherein said lipid component is recovered by:
disrupting said proliferated cell;
extracting said lipid component from the disrupted cell with a solvent; and
recovering said lipid component from the extract.

6. The process of claim 5, wherein said disruption step is effected by homogenization, ultrasonification and/or lyophyilization.

7. The process of claim 5, wherein said solvent is a member selected from the group consisting of chloroform, butanol, isopropanol, ether, acetone, ethanol, methanol, hexane, and mixtures thereof.

8. A pigmentation-lightenihng agent which contains as an effective ingredient a lipid component containing at least either of fatty acid and its derivative, said lipid component being recovered from an established cell line from a warm-blooded animal.

9. The agent of claim 8, wherein said lipid component is obtained by either allowing an established cell line form a warm-blooded animal to proliferate in vitro or allowing the established cell line to proliferate under the supply of a nutrient body fluid of a non-human warm-blooded animal, and
recovering form the resultant cell a lipid component containing at least either of fatty acid and its derivative.

10. The agent of claim 9, wherein said established cell line is a member selected from the group consisting of Namalwa cell (ATCC CRL 1432) and RPMI 1846 cell (ATCC CCL 49).

11. The agent of claim 9, wherein said fatty acid is a member selected from the group consisting of pentadecenoic acid ($C_{15:1}$), oleinoic acid ($C_{18:1}$), linolenic acid ($C_{18:2}$), linolenic acid ($C_{18:3}$), eicosatetraenoic acid ($C_{20:4}$), and mixtures thereof.

12. The agent of claim 9, wherein said fatty acid derivative is a member selected from the group consisting of glycerides and phospholipids of pentadecenoic acid ($C_{15:1}$), oleinoic acid ($C_{18:1}$), linolenic acid ($C_{18:2}$), linolenic acid ($C_{18:3}$), eicosatetraenoic acid ($C_{20:4}$), and mixtures thereof.

13. The agent of claim 9, wherein said lipid component is recovered by:
disrupting said proliferated cell;
extracting said lipid component from the disrupted cell with a solvent; and
recovering said lipid component from the extract.

14. The process of claim 13, wherein said disruption step is effected by homogenization, ultrasonification and/or lyophyilization.

15. The process of claim 13, wherein said solvent is a member selected from the group consisting of chloroform, butanol, isopropanol, ether, acetone, ethanol, methanol, hexane, and mixtures thereof.

16. The agent of claim 9, which is used in the form of medicine or cosmetic.

17. The agent of claim 16, wherein said medicine is a member selected from the group consisting of injection, oral agent, external application, bath liquid, and bath salts.

18. The agent of claim 16, wherein said cosmetic is a member selected from the group consisting of emulsion, pack, cream and lotion.

19. The agent of claim 16, which is used in the preventing and treatment on local and systemic chromatosis.

20. The agent of claim 16, which contains said agent in the range of about 0.001 - 10,000 units per dose.

21. The agent of claim 16, which additionally contains a member selected from the group consisting of antioxidant, cyclodextrin and mixtures thereof.

# FIG. 1

PEAK NO.　　1 → C 15 : 1
　　　　　　　2 → C 18 : 3
　　　　　　　3 → C 20 : 4
　　　　　　　4 → C 18 : 2
　　　　　　　5 → C 20 : 1

HPLC ANALYSIS CONDITION

　　COLUMN:　　　ISK GEL ODS-801m　4.6mm × 15cm
　　MOVING BED:　WATER/ACETONITRILE 60 → 100%
　　　　　　　　　LINEAR GRADIENT
　　VELOCITY:　　0.8ml/min.
　　DETECTION:　　UV 215nm　0.32 FULL SCALE
　　INJECTION:　　10μl